# EUROPEAN PATENT APPLICATION

(11) **EP 3 736 570 A1**
(43) Date of publication of application: **11.11.2020**
(21) Application number: 19382356.4
(22) Date of filing: 09.05.2019
(51) Int. Cl.: G01N 33/50, G01N 33/68

(54) **METHOD FOR THE DIAGNOSIS, SUB-CLASSIFICATION AND PROGNOSIS OF ACUTE KIDNEY INJURY BY DETECTING CCT7**

(71) Applicant: Fundación Instituto de Estudios de Ciencias de la Salud de Castilla y León, 42002 Soria (ES); Universidad de Salamanca (USAL), 37008 Salamanca (ES)
(72) Inventor: LÓPEZ HERNÁNDEZ, Francisco José, 37007 Salamanca (ES); SANCHO MARTÍNEZ, Sandra María, 37007 Salamanca (ES)
(74) Representative: Pons

(57) **Abstract**

The invention provides *in vitro* methods for diagnosing acute kidney injury (AKI), for the prognosis of AKI, for the sub-classification of AKI patients in intrinsic AKI, preferably intrinsic AKI that courses with acute tubular necrosis (ATN) due to renal cortical tubule damage, for monitoring the response to a treatment against AKI in a patient and for testing the nephrotoxic activity of a compound. These methods comprise the quantification of the amount of TCP1-eta protein in urine samples taken from patients.

## Description

This invention belongs to the fields of medicine and nephrology, specifically to methods for the diagnosis, sub-classification and prognosis of patients suffering from acute kidney injury. Particularly, the invention refers to *in vitro* methods which use the quantification of expression levels of a specific biomarker in urine samples for the diagnosis of acute kidney injury and also for the sub-classification of these patients in the sub-group of intrinsic acute kidney injury cursing with acute tubular necrosis due to the presence of renal cortical tubule damage. These methods further allow predicting the progress of the acute kidney injury.

### BACKGROUND ART

Acute kidney injury (AKI) is a syndrome of sudden renal excretory dysfunction with increasing incidence, serious health consequences and high cost. Worldwide, AKI carries an overall mortality of 23.9% in adults and 13.8% in children. In intensive and critical patients mortality may reach 50-80%, especially when AKI occurs in the context of multiorgan failure. A fraction of patients never recover previous renal function. For instance, 7.5% of patients require permanent dialysis after AKI, a number that grows to 40-60% among those with prior chronic kidney disease (CKD). Independently, AKI also increases the odds of progressing to CKD. A worse outcome has been linked to AKI severity, repeated episodes, and prior CKD. But transient and even subclinical AKI also impact on outcome.

Pathogenesis, etiology and AKI type are also associated to prognosis. In this sense, AKI has been traditionally classified into three types, namely pre-renal, renal (or intrinsic) and post-renal, with distinct etiopathology. Pre-renal AKI (i.e. pre-renal azotemia) is believed to be the mildest form. In pre-renal AKI, kidney structures are preserved and, consequently, it is associated to a better clinical outcome than intrinsic AKI, which involves renal parenchymal damage (Kaufman, J., et al., 1991. American Journal of Kidney Diseases: The Official Journal of the National Kidney Foundation 17 (2): 191-98). The commonest pattern of intrinsic AKI is acute tubular necrosis (ATN). ATN is a rather ambiguous term comprising primary and heterogeneous damage forms to the renal tubular compartment, including sublethal alterations in tubule cells compromising tubular function. Prognosis of patients with mild, sublethal alterations may differ substantially from that of patients with extensive tissue destruction. Cataloguing the whole range between both extremes under a unique ATN concept abrogates effective classification and stratification criteria-based care, which is crucial for patient outcome. New diagnostic criteria and biomarkers are necessary to sub-classify the concept of ATN into distinct pathophysiological scenarios.

Etiological diagnosis of AKI and patient stratification are still poorly performed retrospectively, based on the duration of the episode and the response to fluid therapy. In addition, an undetermined amount of pre-renal AKI cases involves a variable degree of renal damage, as an initial stage of a complex continuum, which further complicates diagnosis, and immediate and prospective patient triage on solid rather than on exclusion criteria. Prospectively, a pathophysiological diagnosis based on more specific biomarkers will help to refine prognosis estimation and will enable precision medicine and personalized clinical handling.

The absence of biomarkers sub-stratifying AKI patients into pathophysiological categories, prevent to assign prognosis and differentiated clinical handling. No biomarker has been unambiguously associated to tubule cell death or is able to provide etiological distinction of the disease.

For instance, the gold standard biomarker [i.e. plasma creatinine (Crpl)] conveys no etiological information, as it increases in all forms of AKI (Sancho-Martínez, Sandra M., et al., 2015. New Horizons in Translational Medicine 2(4-5): 110-17).

Biomarkers of subclinical AKI, such as neutrophil gelatinase-associated lipocalin (NGAL), have been proposed to distinguish pre-renal from intrinsic AKI (Vanmassenhove, Jill, et al., 2013. Critical Care 17 (5): R234). Related to this, ES2330005T3 describes a method for the diagnosis of renal tubular cell injury, including an ischemic renal injury and a nephrotoxic injury, wherein the method comprises the detection and quantification of urine NGAL. However, the diagnostic capacity of NGAL has been questioned. In fact, NGAL also increases in the urine as a consequence of systemic or extra-renal inflammation (Oikonomou, K. A., et al., 2012. Journal of Gastroenterology 47(5): 519-30) and different chronic nephropathies (Endre, Zoltan H., et al., 2013. Contributions to Nephrology, 182:30-44), so that it must be considered an unspecific biomarker.

None of these markers, Crpl or NGAL, has shown differential diagnostic capacity among ATN patterns. Accordingly, more specific biomarkers of differentiated pathophysiological scenarios, events, and mechanisms are needed.

With this aim, WO02095000A2 refers to a method of predicting renal tubular injury caused by a compound, such as cisplatin, comprising the assessment of a large gene expression profile. US2007154897A1 discloses urine biomarkers for detecting acute tubular necrosis, wherein said biomarkers are annexin A2 and S100A6 (S100 calcium-binding protein A6). EP3363347A1 describes a method for the early detection of acute kidney injury, comprising the use of measurements of fibroblast growth factor 23 (FGF23), klotho (KL) and erythropoietin (EPO) as biomarkers, where such measurements are obtained from a venous blood sample or urine. EP2511704A1 relates to a method of diagnosis of prerenal and intrinsic acute kidney injury and to equipment and kits for use in this method. Said method comprises the step of assessing the calprotectin level in a urine sample taken from a patient suspected of suffering from intrinsic acute kidney injury. Finally, Won K. Han, *et al.* (Won K. Han, et al., 2002. Kidney International 62: 237-244) described increased levels of KIM-1 (Kidney Injury Molecule-1) in urine of patients suffering from ischemic ATN, thus proposing KIM-1 as a biomarker for ATN in patients with AKI.

However, identifying alternative, specific biomarkers involved in distinct pathophysiological scenarios of AKI is still crucial in the field of nephrology, since they will allow an accurate sub-classification of AKI patients. In particular, it would be especially interesting to identify biomarkers for intrinsic AKI cursing with ATN due to the presence of renal cortical tubule injury, since these patients show a worse prognosis and their identification is therefore crucial in order to initiate an early suitable therapy. These biomarkers will help to stablish an individualized prognosis or clinical outcome of the disease and, consequently, it will enable to personalize and implement the most suitable therapy to be administered to each patient.

### DESCRIPTION OF THE INVENTION

In this invention, tail-less complex polypeptide-1 eta subunit (TCP1-*eta*) protein has been identified as a specific urinary biomarker of AKI and, more specifically, of renal cortical tubule injury which is associated to a defective recovery, i. e. to a bad prognosis. Therefore, this biomarker allows, not only the diagnosis of AKI, but also it contributes to the pathophysiological sub-classification of AKI for a more accurate patient stratification and personalized handling.

The present invention provides non-invasive methods and uses of a kit for quantifying the amount of TCP1-*eta* protein in urine samples taken from patients and identifying the presence of AKI, and particularly the presence of ATN due to renal cortical tubule damage which is associated to a defective recovery.

In examples shown in this description, it is evidenced that TCP1-*eta* is present in the supernatant of tubular cells undergoing apoptosis, and subsequently in the urine of rats with ATN in correlation with the extent of renal cortical damage, and of AKI patients in correlation to a bad prognosis (i.e. incomplete recovery from AKI).

Inventors of the present invention used a cell-based system to identify TCP1-*eta* in the culture medium as a marker of damaged renal tubule cells. In rat models of AKI, TCP1-*eta* is increased in the urine co-relating with renal cortical tubule damage. Urinary TCP1-*eta* is found in etiologically heterogeneous AKI patients, but it is statistically higher in patients not fully recovering from severe AKI. Consequently, urinary TCP1-*eta* is a specific sub-stratifying biomarker of renal cortical damage associated to bad prognosis.

Therefore, one of the main advantages of the present invention is that it uses a biomarker whose quantification may be performed in a non-invasive way. Expression levels or amount of TCP1-*eta* can be easily quantified since this protein is secreted by kidney cells into the urine. Furthermore, this invention allows an accurate stratification or sub-classification of AKI patients in intrinsic AKI cursing with ATN due to the presence of renal cortical tubule damage. This particular sub-group of AKI patients shows a worse prognosis and does not recover or does not fully recover, so that these subjects must be early diagnosed in order to initiate a suitable individualized clinical handling as soon as possible. The methods proposed by the invention satisfy this need.

In view of the above, one aspect of the invention refers to the use of the expression levels of urine TCP1-*eta* as a quantitative biomarker for the *in vitro* diagnosis of acute kidney injury.

"Acute kidney injury" (AKI) is defined as an abrupt (within approximately 48 hours) decrease in kidney function, based on an elevation in serum creatinine level, a reduction in urine output, the need for renal replacement therapy (dialysis) or a combination of these factors. It may encompass both injury (structural damage) and impairment (loss of function). It is a syndrome that rarely has a sole and distinct pathophysiology. Many patients with AKI have a mixed etiology where the presence of sepsis, ischemia and nephrotoxicity often co-exist and complicate recognition and treatment. Furthermore, the syndrome is quite common among patients without critical illness and it is essential that health care professionals, particularly those without specialisation in renal disorders, detect it easily. AKI is classified in three types, named pre-renal, renal (or intrinsic) and post-renal. The term "AKI" as used in this invention includes these three types. Pre-renal AKI is caused by decreased renal perfusion, often because of volume depletion, intrinsic AKI is caused by a process within the kidneys, and post-renal AKI is caused by inadequate drainage of urine distal to the kidneys. Of these, 'intrinsic' AKI represents true kidney disease, while pre-renal and post-renal AKI are the consequence of extra-renal diseases leading to the decreased glomerular filtration rate (GFR). If these pre- and/or post-renal conditions persist, they will eventually evolve to renal cellular damage and hence intrinsic renal disease.

"TCP1" is a chaperonin-containing, hetero-oligomeric complex formed by eight subunits, including *eta* subunit. TCP1 is known to contribute to actin and tubulin folding in the cytosol, and thus to the conformation of cytoskeleton, cell shape and cell division. Tyrosine phosphorylation of TCP1 is also involved in cell detachment during apoptosis. "T-complex protein 1 subunit eta" (TCP1-*eta* or CCT-*eta*) preferably refers, in the present invention, to the human protein found in UniProtKB database under the accession number Q99832, or any fragment thereof. The gene encoding this TCP1-*eta* protein is named CCT7. In this invention, within the term TCP1-*eta* are encompassed the four isoforms Q99832-1, Q99832-2, Q99832-3 and Q99832-4 described in the UniProtKB database.

The term "expression levels" refers to expression levels of both a transcriptional or translational product of the gene, i.e. to mRNA or protein expression levels. Expression levels referred to throughout the present description and claims are, preferably, TCP1-*eta* protein levels/amount.

The term "quantitative biomarker" refers to the amount or concentration of mRNA or protein used as an indicator useful for the diagnosis, sub-classification and/or prognosis.

The term "diagnosis" refers to the process performed in order to identify the presence or absence of a pathological condition, particularly AKI, preferably intrinsic AKI, more preferably intrinsic AKI that courses with ATN due to renal cortical tubule damage, in a subject, preferably in a human subject.

Another aspect of the invention refers to the use of the expression levels of urine TCP1-*eta* as a quantitative biomarker for the *in vitro* prognosis and sub-classification of AKI patients in intrinsic AKI, preferably intrinsic AKI that courses with acute tubular necrosis (ATN) due to renal cortical tubule damage, which is associated to a bad prognosis. Alternatively, this use refers to the *in vitro* diagnosis of intrinsic AKI, preferably intrinsic AKI that courses with ATN due to renal cortical tubule damage.

The term "prognosis" refers to the process performed in order to predict the events that will occur during the curse of a pathological condition, particularly AKI, preferably intrinsic AKI, more preferably intrinsic AKI that courses with ATN due to renal cortical tubule damage, including recovery or non-recovery from the pathological condition to a health state showed prior to the AKI episode.

The term "sub-classification" is considered to be the procedure through which a subject suffering from AKI, thus belonging to the group of AKI patients, is assigned to a subgroup of a specific AKI form, preferably to the subgroup of intrinsic AKI, more preferably intrinsic AKI that courses with ATN due to renal cortical tubule damage associated to a bad prognosis.

"Intrinsic AKI" can be categorized by the component of the kidney that is primarily affected (i.e., tubular, glomerular, interstitial, or vascular). "Acute tubular necrosis" (ATN) is a common type of intrinsic AKI and it is usually caused by ischemia (from prolonged hypotension) or nephrotoxic insults (from an agent that is toxic to the tubular cells). In contrast to a pre-renal etiology, intrinsic AKI caused by ATN does not improve with adequate repletion of intravascular volume and blood flow to the kidneys. Both ischemic and nephrotoxic ATN can resolve over time, although temporary renal replacement therapy may be required, depending on the degree of renal injury. In other cases, ATN is not resolved and patients show a permanent impaired renal function which may even result in a progressive and irreversible chronic renal disease. In the most severe cases, ATN patients need dialysis indefinitely.

The present invention allows the sub-classification of AKI patients in intrinsic AKI, versus pre-renal and post-renal AKI, and further in intrinsic AKI specifically due to the occurrence of a renal cortical tubule injury.

In a preferred embodiment, the intrinsic AKI referred to in the present invention is caused by an ischemia/reperfusion injury or by the exposure to a nephrotoxic agent for instance, a nephrotoxic drug such as cisplatin or gentamicin.

Another aspect of the invention, refers to the use of the expression levels of urine TCP1-*eta* as a quantitative biomarker for the *in vitro* monitoring of the response to a treatment or the effectiveness of a treatment in a patient suffering from AKI, preferably intrinsic AKI, more preferably intrinsic AKI that courses with ATN due to renal cortical tubule damage.

Another aspect of the invention, refers to the use of the expression levels of TCP1-eta as a quantitative biomarker for the *in vitro* testing of the nephrotoxic activity or renal toxicity of a compound, composition, agent, molecule or drug.
Examples of nephrotoxic compounds/agents are, without limitations, cephaloridine, cisplatin, puromycin aminonucleoside (PAN), bromoethylamine hydrobromide (BEA), gentamicin, ifosfamide, cyclophosphamide, carboplatin, AY-25329, indomethacin, acyclovir, citrinin, mercuric chloride, diflunisal, cidofovir, pamidronate, lithium, hydralazine, colchicine, sulfadiazine, cycloheximide, iodinated contrast media (such as iohexol), proton pump inhibitors (such as omeprazole) and adriamycin.

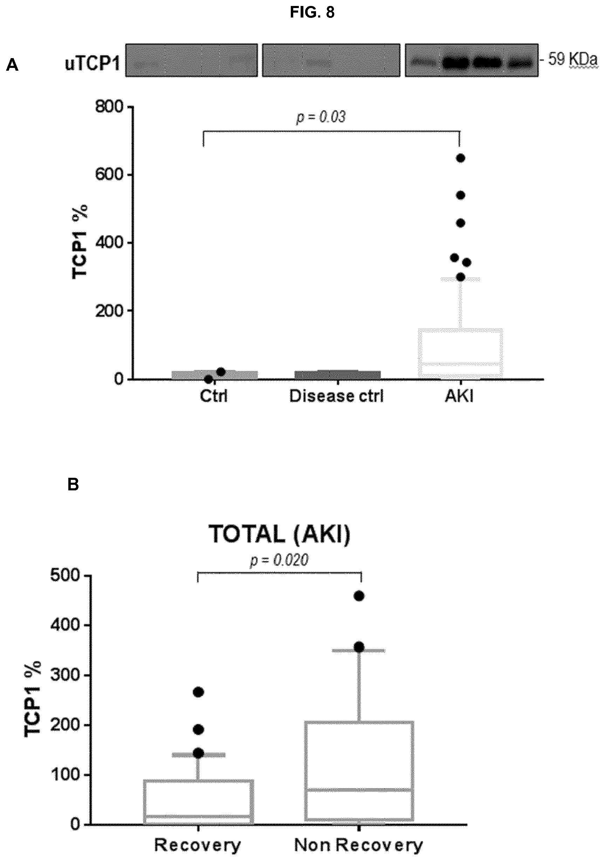

Examples accompanying the present description evidence that urine taken from AKI patients shows higher levels of the TCP1-*eta* biomarker than individuals non-suffering from AKI (see Fig. 8A). Thus, another aspect of the invention refers to an *in vitro* method, hereinafter "the first method of the invention", for the diagnosis of AKI in a subject, wherein said method comprises the following steps:
a. quantifying the amount of urine TCP1-*eta* protein in an isolated urine sample taken from a subject,
b. comparing the value obtained in step (a) to a standard value obtained from the quantification of the amount of urine TCP1-*eta* protein in a urine sample isolated from a healthy subject, and
c. assigning the subject of step (a) to the group of patients suffering from AKI when the value obtained after the quantification of step (a) is significantly higher than the standard value.

The expression "to quantify" the amount of urine TCP1-*eta* protein in an isolated urine sample, as used in this invention, refers to the measurement of the amount or the concentration of protein in the sample.

The term "amount" as used herein refers, but without limitation, to the absolute or relative amount of TCP1-*eta* protein, preferably expressed as arbitrary units or percentage (%), as well as any other value or parameter related to this or that can be derived from this. These values or parameters comprise values of signal intensity obtained from any physical or chemical property of this protein obtained by direct measurement, for example, values of intensity of mass spectroscopy or nuclear magnetic resonance. Additionally, these values or parameters include all those obtained through indirect measurement.
Preferably, the amount of urine TCP1-*eta* protein is measured by a technique selected from *in situ* hybridization using specific labeled anti-TCP 1-*eta* antibodies or immunologically active fragments thereof, electrophoresis gels, membrane transfer and hybridization with a specific probe, MRI or any other image diagnosis technique using paramagnetic nanoparticles or other type of functionalized nanoparticles, assays such as Western blot, immunoprecipitation assays, protein arrays, preferably antibodies based microarrays, flow cytometry, immunofluorescence, immunohistochemistry, chemiluminescence, multiplex bead assay, immunoassays such as enzyme-linked immunosorbent assay (ELISA), radioimmunoassay (RIA), or any other enzymatic method, by incubation with a specific ligand, or chromatographic techniques preferably combined with mass spectrometry, spectroscopy, mass spectrometry, including SELDI mass spectrometry, colorimetry, electrophoresis or isoelectric focusing.

For immunoassays, anti-TCP1-*eta* antibodies or immunologically active fragments thereof used may be labeled and/or immobilized in a support such as a microtiter plate or bio-chip. Suitable labels are, for instance, secondary antibodies, an enzyme, radioisotopes, magnetic tags or fluorescence or the like.

An "immunoassay" or "immunochemical assay" is a biochemical assay that detects and/or quantifies the amount or quantity (preferably concentration) of one or more peptides of interest (in the context of the present invention, the TCP1-*eta* protein) in a sample. For this purpose, the reaction uses one or more antibodies specific for the antigen/s to be detected. The quantification of the protein to be detected may be performed by any of the methods known in the art, such as the labeling of the antibody/ies or the antigen/s. The immunoassay may be competitive or non-competitive. In a competitive immunoassay the signal detected will be inversely proportional to the concentration of antigen in the sample. In a non-competitive assay (such as an "ELISA sandwich assay") the signal detected is directly proportional to the concentration of antigen in the sample.

Examples of immunoassays useful for performing the methods of the invention are, but not limited to: immunoblotting (Western blot), enzyme-linked immunosorbent assay (ELISA), multiplex bead assay, dipstick assay, line immunoassay (LIA), radioimmunoassay (RIA), immunoradiometric assay (IRMA), immunofluorescence, chemiluminescence, passive haemagglutination, immunogold labeling (transmission electron microscopy), x-map or protein or lipopolysaccharide chips (LPS), real-time immunoquantitative PCR (iqPCR), electrochemiluminescent tags, label-free immunoassays (i.e. surface plasmon resonance, etc.), photoacoustic immunoassay, cloned enzyme donor immunoassay (CEDIA), lateral flow immunochromatographic assays, magnetic immunoassay (MIA), surround optical-fiber immunoassay (SOFIA), compact disk/digital versatile disk (CD/DVD) based immunoassay, or agglutination-PCR (ADAP).

More preferably, the amount of urine TCP1-*eta* protein is measured/quantified in the methods described in the present invention by immunoassay, even more preferably by ELISA or Western blot.

ELISA is based on the assumption that an immunoreagent (antigen or antibody) can be immobilized onto a solid support, then this system is put in contact with a fluid phase containing the complementary reagent that can be bound to a marker compound (label). The ELISA technique referred to in the present invention may be a direct ELISA, indirect ELISA or sandwich ELISA. The signal produced as a consequence of the labeling reaction with the antibody conjugated with a detection system may be measured in the present invention, for instance but without limitation, by spectrophotometry, chemiluminiscence detection and quantification, spectrofluorometric detection and quantification, bioluminescence, differential calorimetry, analytical ultracentrifugation, interferometry, etc., preferably by chemiluminiscence detection and quantification.

The amount of products/substrates related to the activity of the TCP1-*eta* protein may be alternatively measured in the methods described herein as undirect measurement of the amount of biomarker present in the urine sample.

The term "standard value" or "threshold value", as used in the first method of the invention, is any value or range of values derived from the quantification of the amount of urine TCP1-*eta* protein in a urine sample isolated from a healthy subject or in a mixture of urine samples isolated from healthy subjects.
By "healthy subject" is meant an individual who does not suffer from AKI.

The healthy subject or group of healthy subjects chosen for obtaining the standard value for performing the first method of the invention, must have the same or similar age as the subject under study and are representative of the population in which the first method of the invention is to be applied. The quantification must be made in the same way as that for the subject to be studied in step (a) of the first method of the invention.

The term "comparison" as used in this invention refers, but is not limited to, the comparison of the amount of urine TCP1-*eta* protein determined in the urine sample of the subject under study with the standard value. The comparison described in the methods of the invention can be performed manually or computer-assisted.

An amount which is "significantly higher or lower" than a standard value can be established by an expert in the field through the use of various statistical tools such as, for example but without limitation, by determination of confidence intervals, determination of the p value, two-tailed unpaired Student's t test, Fisher's discriminant functions, using Kruskal-Wallis one way analysis with Dunn's multiple comparisons test, one-way ANOVA with Bonferroni's post hoc multiple comparisons test, two tailed Mann-Whitney U test or Kruskal-Wallis U tests.

Another aspect of the invention refers to an *in vitro* method, hereinafter "the second method of the invention", for the prognosis and sub-classification of patients suffering from AKI in intrinsic AKI, preferably intrinsic AKI coursing with ATN due to renal cortical tubule damage, wherein said method comprises the following steps:
a. quantifying the amount of urine TCP1-*eta* protein in an isolated urine sample taken from a patient suffering from AKI,
b. comparing the value obtained in step (a) to a standard value obtained from the quantification of the amount of urine TCP1-*eta* protein in a urine sample isolated from a subject suffering from pre-renal AKI, and
c. assigning the patient of step (a) to the group (sub-group) of patients suffering from intrinsic AKI, preferably intrinsic AKI coursing with ATN due to renal cortical tubule damage, associated to a bad prognosis, when the value obtained after the quantification of step (a) is significantly higher than the standard value.

The term "standard value" or "threshold value", as used in the second method of the invention, is any value or range of values derived from the quantification of the amount of urine TCP1-*eta* protein in a urine sample isolated from a subject suffering from pre-renal AKI or mixture of urine samples isolated from subjects suffering from pre-renal AKI.

"Pre-renal AKI" is a subtype of AKI in which underlying kidney function may be normal, but decreased renal perfusion associated with intravascular volume depletion (e.g., from vomiting or diarrhea) or decreased arterial pressure (e.g., from heart failure or sepsis) results in a reduced glomerular filtration rate. Autoregulatory mechanisms often can compensate for some degree of reduced renal perfusion in an attempt to maintain the glomerular filtration rate. Several medications can cause pre-renal AKI. Notably, angiotensin-converting enzyme inhibitors and angiotensin receptor blockers can impair renal perfusion by causing dilation of the efferent arteriole and reduce intraglomerular pressure. Nonsteroidal anti-inflammatory drugs also can decrease the glomerular filtration rate by changing the balance of vasodilatory/vasoconstrictive agents in the renal microcirculation. These drugs and others limit the normal homeostatic responses to volume depletion and can be associated with a decline in renal function. In patients with pre-renal AKI, kidney function typically returns to baseline after adequate volume status is established, the underlying cause is treated, or the offending drug is discontinued. Some of the physical symptoms associated to pre-renal AKI are, for instance, weight loss, orthostatic hypotension, tachycardia, poor skin turgor, dilated neck veins, pulmonary rales, peripheral edema, ascites, caput medusae, and/or spider angiomas.

Preferably, the intrinsic AKI referred to in the second method of the invention is caused by an ischemia/reperfusion injury or by the exposure of a nephrotoxic agent for instance, a nephrotoxic drug such as cisplatin or gentamicin.

A "good prognosis" or "good recovery" means that renal function (evaluated as plasma creatinine concentration) returns to normal (healthy) values or values prior to the AKI.

"Bad prognosis", "bad recovery" or "bad outcome" refers, in the present invention, to non-recovery or incomplete recovery from AKI to baseline kidney function showed prior to the disease. Thus, a bad prognosis refers to those cases in which renal function (assessed as plasma creatinine concentration) does not return to normal (healthy) or previous values (i.e. values prior to the AKI).

Once an intervention and/or treatment against AKI, preferably intrinsic AKI, more preferably intrinsic AKI that courses with ATN due to renal cortical tubule damage, has commenced, the amount of the urine TCP1-*eta* protein may be quantified in order to monitor the progress and efficacy of the treatment or intervention. Thus, another aspect of the invention refers to an *in vitro* method, hereinafter "the third method of the invention", for monitoring the response to a treatment or the effectiveness of a treatment in a patient suffering from AKI, preferably intrinsic AKI, more preferably intrinsic AKI that courses with ATN due to renal cortical tubule damage, wherein said method comprises the following steps:
a. quantifying the amount of (basal) urine TCP1-*eta* protein in an isolated urine sample taken from the patient prior to the administration of the treatment,
b. quantifying the amount of urine TCP1-*eta* protein in an isolated urine sample taken from the same patient after the administration of the treatment,
c. comparing the value obtained in step (a) and the value obtained in step (b), and
d. assigning the patient of step (a) to the group of patients showing a good response to the treatment when the value obtained after the quantification of step (b) is significantly lower than the value obtained after the quantification of step (a).

One or more post-treatment urine samples may be taken and analyzed in step (b) of this method for quantifying the amount of urine TCP1-*eta* protein as the treatment commences and continues. Thus, several urine samples may be taken, at different time points as the treatment continues, and analyzed according to steps (b) to (d) of the third method of the invention. This is useful to check how the patient is responding to the therapy over the time.

The subject or patient referred to in the methods of the invention may be a human, but also a non-human mammal such as, for example but without limitation, rodents, ruminants, felines or dogs. Therefore, in another preferred embodiment, the subject or patient of the methods of the invention is a mammal. In a more preferred embodiment of the methods of the invention, the subject or patient is a human.

In all the methods described in this invention, an agent specific for total protein or a normalizing protein, such as creatinine, may also be used, or an assay to measure the level or concentration of total protein or a normalizing protein may be performed in order to normalize the level or concentration of TCP1-*eta* protein. Such normalization is often useful in order to permit various comparisons of urine TCP1-eta levels, or determine ratios, for example, between subject samples, or between a series of samples isolated from the same subject at different timepoints (e.g., at a first timepoint and/or between any subsequent timepoint thereafter).

In the methods of the invention, an additional step may be performed that comprises selecting the subject or patient for an appropriate treatment for intrinsic AKI, preferably intrinsic AKI coursing with ATN due to renal cortical tubule damage.

In this invention, it has been studied the supernatant of tubule cells cultured with nephrotoxic drugs for released biomarkers which could be eventually found in the urine following a tubular injury. As shown in examples below (see Examples 1 and 2), cultured mouse cortical tubule cells (MCT cells) exposed to nephrotoxic compounds, such as cisplatin or cycloheximide, increased their TCP1-*eta* secretion to the culture medium. Therefore, TCP1-*eta* is further useful as a biomarker of cytotoxicity *in vitro* and, accordingly, the measurement of the TCP1-*eta* protein or gene expression levels in an *in vitro* cell culture previously administered with a compound to be tested is useful for analysing the nephrotoxic activity of said compound.

Therefore, another aspect of the invention refers to an *in vitro* method, hereinafter "the fourth method of the invention", for testing the nephrotoxic activity or renal toxicity of a compound, composition, agent or drug, wherein said method comprises the following steps:
a. *in vitro* culturing cells and quantifying the expression levels of TCP1-*eta* gene or the amount of TCP1-*eta* protein secreted to the culture medium prior to the administration of the compound to be tested,
b. administering the compound to be tested to the cells in culture of step (a),
c. quantifying the expression levels of TCP1-*eta* gene or the amount of TCP1-*eta* protein secreted to the culture medium after the administration of the compound to be tested in step (b),
d. comparing the value obtained in step (a) and the value obtained in step (c), and
e. identifying the compound as nephrotoxic when the value obtained after the quantification of step (c) is significantly higher than the value obtained after the quantification of step (a).

In a preferred embodiment of the fourth method of the invention, cultured cells are derived from kidney tissue, more preferably they are cortical tubule cells.

In another preferred embodiment of the fourth method of the invention, cultured cells are from human origin.

Techniques for quantifying the expression levels of TCP1-*eta* gene are, for example but without limitation, PCR, RTLCR, RT-PCR, semi- or quantitative real time PCR (qRT-PCR), digital PCR (dPCR), Southern Blot, Northern Blot, dot or slot blot, nuclease protection, branched-chain DNA or any other method for the amplification of nucleic acids; DNA microarrays made with oligonucleotides deposited by any technique, DNA microarrays made with in situ synthesized oligonucleotides, *in situ* hybridization using specific labeled probes, electrophoresis gels, membrane transfer and hybridization with a specific probe, MRI or any other image diagnosis technique using paramagnetic nanoparticles or other type of functionalized nanoparticles. Preferably, the expression levels of TCP1-*eta* gene are quantified in the present invention by RT-qPCR.

Another aspect of the invention refers to a method for testing the nephrotoxic activity or renal toxicity of a compound, composition, agent or drug, wherein said method comprises: (a) *in vivo* administering the compound, composition, agent or drug to be tested to a living animal, for instance a laboratory rat, (b) quantifying the amount of urine TCP1-*eta* protein in a urine sample isolated from the animal after the administration of step (a), (c) comparing this value with an standard value, wherein the standard value is the amount of urine TCP1-*eta* protein quantified in a urine sample isolated from the same animal before the administration of the compound, composition, agent or drug to be tested, and (d) identifying the compound, composition, agent or drug as nephrotoxic when the value obtained after the quantification of step (b) is significantly higher than the standard value.

Another aspect of the invention refers to a kit, hereinafter "the kit of the invention", comprising probes, primers and/or antibodies specific for (i. e. that specifically hybridize with or bind to) TCP1-*eta* gene or protein or a fragment thereof. Preferred primers to be included in this kit are those of SEQ ID NO: 1 and SEQ ID NO: 2.

Kits referred to in the present invention may combine, in different combinations, oligonucleotides or primers, probes, antibodies, reagents, signal detection and array-processing instruments, and the like. These kits may also comprise means for acquiring a quantity of a urine sample from the subject or patient, wherein the acquiring means comprise for instance a container for accepting the urine sample.

In some embodiments, the kits comprise a carrier, package, or container such as bottles, vials, syringes, and test tubes. In other embodiments, the containers are formed from a variety of materials such as glass or plastic.

In some embodiments, the kits comprise one or more additional containers, each with one or more of various materials (such as reagents, optionally in concentrated form, and/or devices) desirable from a commercial and user standpoint for use in the methods as described herein. Non-limiting examples of such materials include, but not limited to, buffers, solutions, primers, enzymes, diluents, filters, carrier, package, container, vial and/or tube, labels, listing contents and/or instructions for use and package. A set of instructions for performing the methods described herein is optionally included in the kit. In some embodiments, the containers comprised in the kit include an identification label. This identification label is preferably used to indicate that the contents of the kit are to be used for a specific diagnostic and/or prognostic application in AKI. This identification label may also indicate guidelines for use of the contents of the kit, such as in the methods described herein.

As used herein a "probe" is defined as a nucleic acid capable of binding to a target nucleic acid of complementary sequence through one or more types of chemical bonds, usually through complementary base pairing, usually through hydrogen bond formation. As used herein, a probe may include natural (i. e., A, G, U, C, or T) or modified bases (such as 7-deazaguanosine, inosine, etc.). In addition, the bases in probes may be joined by a linkage other than a phosphodiester bond, so long as it does not interfere with hybridization. Thus, probes may be peptide nucleic acids in which the constituent bases are joined by peptide bonds rather than phosphodiester linkages. The term "probe", as used in this invention, preferably refers to a small DNA fragment labelled with fluorescence that emits detectable signals and is used as a tool in, for example but without limitation, RT-qPCR to quantify the expression levels of the *TCP1-eta* gene in a sample.

Probes based on the sequence of the *TCP1-eta* gene may be prepared by any commonly available method. See for instance WO 99/32660 for methods of producing probes for a given gene. In addition, any available software may be used to produce specific probe sequences, including, for instance, software available from Molecular Biology Insights, Olympus Optical Co. and Biosoft International. Probes may be produced from any region of the *TCP1-eta* gene. Oligonucleotide probes for assaying a biological sample are preferably of sufficient length to specifically hybridize only to appropriate, complementary gene or transcript. Preferably, the oligonucleotide probes will be at least about 10, 12, 14, 16, 18, 20 or 25 nucleotides in length. In some cases, longer probes of at least 30, 40, or 50 nucleotides will be desirable.

In a preferred embodiment, the kit will also include one or more control probes and/or antibodies. In addition to test probe/s and antibody/ies that bind the target nucleic acid or protein of interest, the kit can contain a number of control probes and/or antibodies. The control probes/antibodies may fall into three categories: 1) normalization controls, 2) expression level controls, and 3) mismatch controls. Expression level controls are probes/antibodies that hybridize specifically with constitutively expressed genes/proteins in the biological sample. Virtually, any constitutively expressed gene/protein provides a suitable target for expression level controls. Typically, expression level control probes/antibodies have sequences complementary or that specifically bind to subsequences of constitutively expressed "housekeeping genes" including, but not limited to, actin, transferrin receptor, GAPDH, ribosomal protein S13 (RPS13), and the like.

As used herein, "primers" are oligonucleotide sequences that are complementary to the *TCP1-eta* gene and are capable of hybridizing under stringent conditions to at least part of the nucleotide sequence of said gene. Primers serve as a starting point for the amplification of DNA. They contain a free 3' hydroxyl group that forms complementary base pairs to the template strand and acts as a starting point for the addition of nucleotides in order to copy and amplify the template strand. Such hybridizable oligonucleotides will typically exhibit at least about 75% sequence identity at the nucleotide level to said gene, preferably about 80% or 85% sequence identity or more preferably about 90% or 95% or more sequence identity to said gene. Preferably, primers referred to in the present invention are those described in SEQ ID NO: 1 and SEQ ID NO: 2.

"Hybridizing" refers to complementary hybridization between a probe or primer nucleic acid and a target nucleic acid and embraces minor mismatches that can be accommodated by reducing the stringency of the hybridization media to achieve the desired detection of the target polynucleotide sequence.

The antibodies, primers and/or probes comprised in the kit may be labelled and/or immobilized, or both. Preferably, they are labelled with a label selected from a radioisotope, fluorescent or luminescent marker, antibody, antibody fragment, affinity label, enzyme or enzyme substrate. More preferably, the antibodies, primers and/or probes are immobilized in a support comprised within the kit. For instance, they are immobilized in each of the wells of a microtiter plate comprised in the kit. Other examples of supports are a nylon or latex matrix, a membrane, a glass support, a plastic support, beads, array and a silicon support. Any solid surface to which oligonucleotides, probes or antibodies can be bound, either directly or indirectly, either covalently or non-covalently, can be used. Furthermore, the solid support surface may be in the format of a dipstick, a test strip, paper-based assay, a latex bead, a microsphere, or a multi-well plate.

Both monoclonal or polyclonal antibodies that bind TCP1-*eta* protein or a fragment thereof are useful in the methods and kits of the present invention. The antibodies can be prepared by methods well-known in the art or may be purchased, since anti-TCP1-*eta* antibodies are commercially available.

Antibodies used in the methods of the invention may be human antibodies, humanized antibodies, recombinant antibodies, monoclonal antibodies, chimeric antibodies, aptamers, peptide or analogues, or conjugates or fragments thereof, a Fab fragment, a F(ab')2 fragment, a Fab' fragment, a F sc fragment, or a Fv fragment.

In another preferred embodiment, the kit further comprises a secondary antibody which preferably binds to a first anti-TCP1-*eta* antibody and more preferably it is conjugated with a detection system. Detection systems to which the first or secondary antibody may be conjugated are those known in the state of the art, preferably, it is the enzyme HRP.

In a more preferred embodiment, the kit of the invention further comprises reactive sticks, fluorochrome/s, dye/s, substrate/s specific for the detection system selected to which the antibodies comprised in the kit are conjugated (these substrates are required for initiating the labeling reaction), blocking buffers, washing solutions and/or stop solutions.

The kit of the invention may also comprise all the elements needed for carrying out the methods as described herein. These additional elements may include, but without limitations, agents and/or proteins to prevent the contamination of the samples and/or elements comprised in the kit, or peptides and/or antibodies that act as positive or negative controls.
Another aspect of the invention refers to the use of a kit comprising primers, antibodies and/or probes specific for (i. e. that specifically hybridize with or that bind to) TCP1-*eta* gene or protein for the *in vitro* diagnosis of AKI, for the *in vitro* prognosis and sub-classification of AKI patients in intrinsic AKI, preferably intrinsic AKI coursing with ATN due to renal cortical tubule damage, associated to a bad prognosis, for the *in vitro* monitoring of the response to a treatment or the effectiveness of a treatment in a patient suffering from AKI and/or for the *in vitro* testing of the nephrotoxic activity of a compound, according to the methods described above. Preferably, this kit is the kit of the invention described above.

Unless otherwise defined, all technical and scientific terms used herein have the same meaning as commonly understood by one of ordinary skilled in the art to which this invention belongs. Methods and materials similar or equivalent to those described herein can be used in the practice of the present invention. Throughout the description and claims the word "comprise" and its variations are not intended to exclude other technical features, additives, components, or steps. Additional objects, advantages and features of the invention will become apparent to those skilled in the art upon examination of the description or may be learned by the practice of the invention. The following examples, drawings and sequence listing are provided by way of illustration and are not intended to be limiting of the present invention.

### DESCRIPTION OF THE DRAWINGS

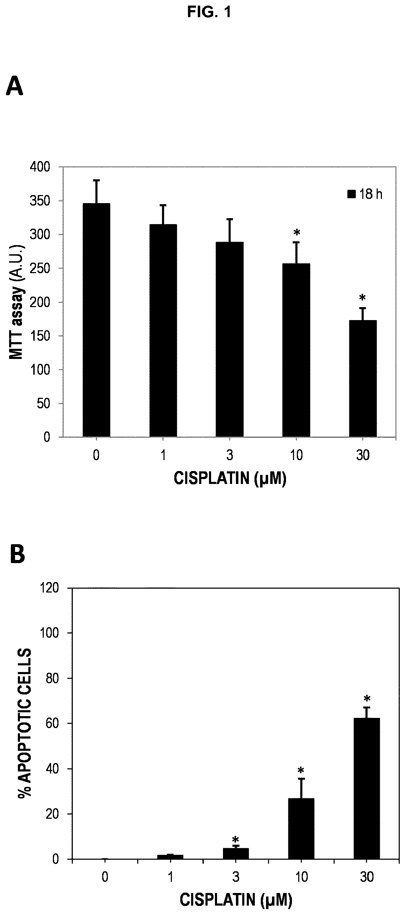

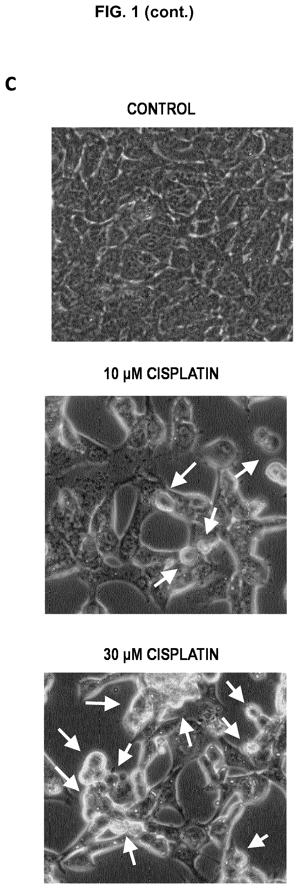

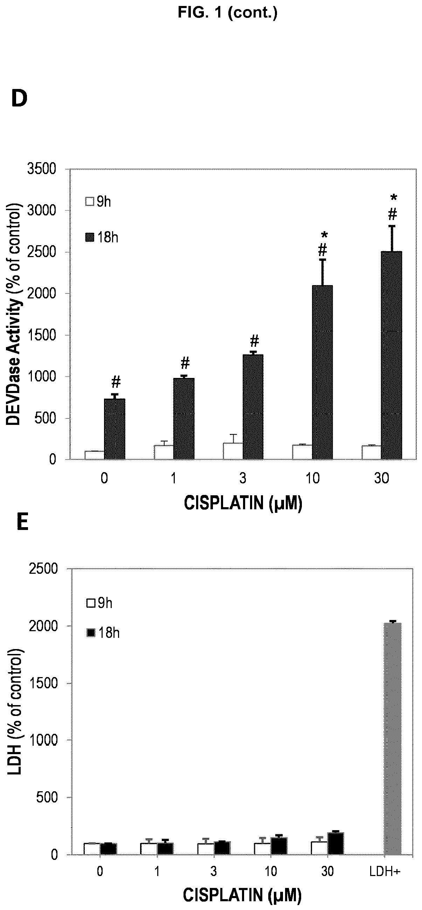

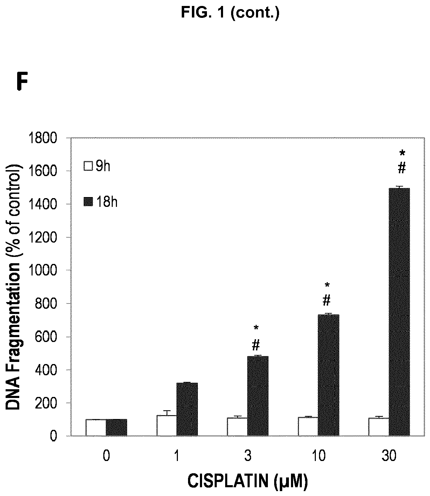

**Figure 1****. Antiproliferative effect and morphological phenotypes induced by cisplatin.** (A) MTT-based proliferation/viability, concentration-effect profile of MCT treated for 18 hours with cisplatin (0-30 µM). Data represent the average ± SD of n=3. *, p<0.05 with respect to control. (B) Quantification of the number of apoptotic cells after 18 hours of treatment with cisplatin (0-30 µM). (C) Representative light microscopy photographs (n= 3, 400x) of MCT cells treated with 0 (control), 10 and 30 µM cisplatin for 18 hours. White arrows: apoptotic cells. (D) DEVDase (caspase) activity in cell extracts, (E) LDH activity in culture medium, (F) Internucleosomal DNA fragmentation. Data represent the average ± SD of n=3. *, p<0.05 with respect to 0 µM cisplatin in its group; #, p<0.05 with respect to the same concentration of cisplatin after 9 hours of treatment.

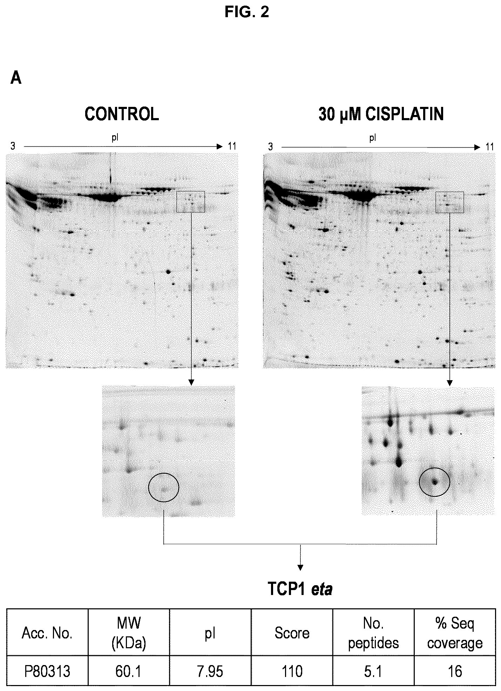

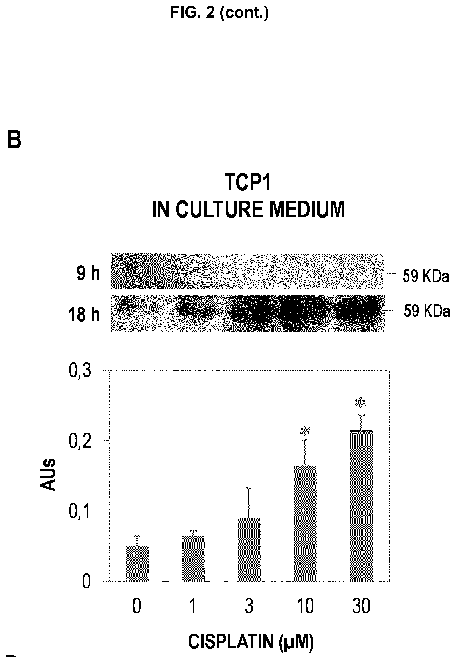

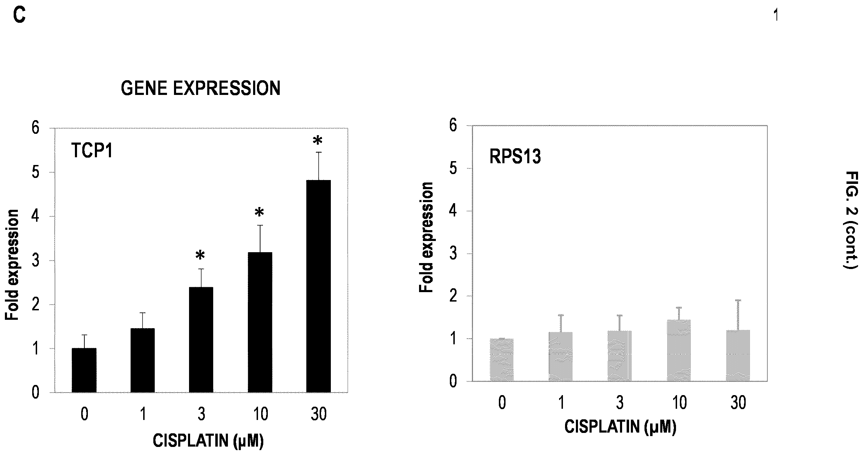

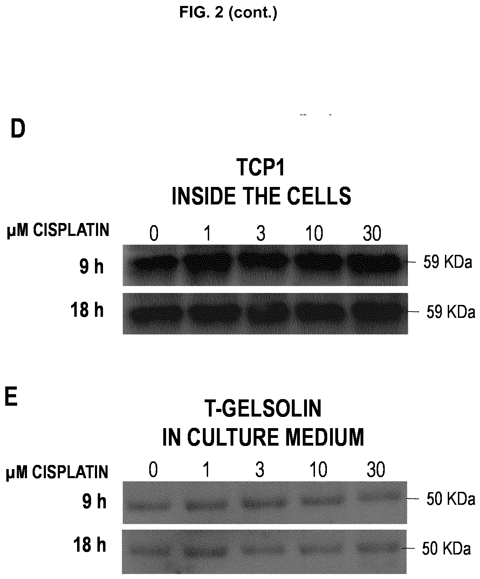

**Figure 2****. Differential proteomic profiling of the culture medium from control and cisplatin-treated cells.** (A) Representative images of 2-DE gels, n=3. The marked spots correspond to proteins significantly increased in the culture medium of MCT cells after treatment for 18 hours with 3 or 30 µM cisplatin with respect to the control. (B) Representative image of Western blot analysis of TCP1-*eta* in culture medium, after 9 and 18 hours, in MCT cells treated with cisplatin (0-30 µM). Data represent the average ± SD of n=3 independent experiments; *, p<0.05 with respect the control. (C) Expression of TCP1-*eta* (left panel) and RPS13 (right panel) mRNA in MCT cells treated with 0-30 µM cisplatin for 18 hours (n=3). Data are expressed as the average ± SD. *, p<0.01 vs control. (D) Representative image of Western blot analysis of TCP1-*eta* inside the cells. (E) Representative image of Western blot analysis of t-Gelsolin in the culture medium; in MCT cells treated with cisplatin (0-30 µM) for 9 and 18 hours.

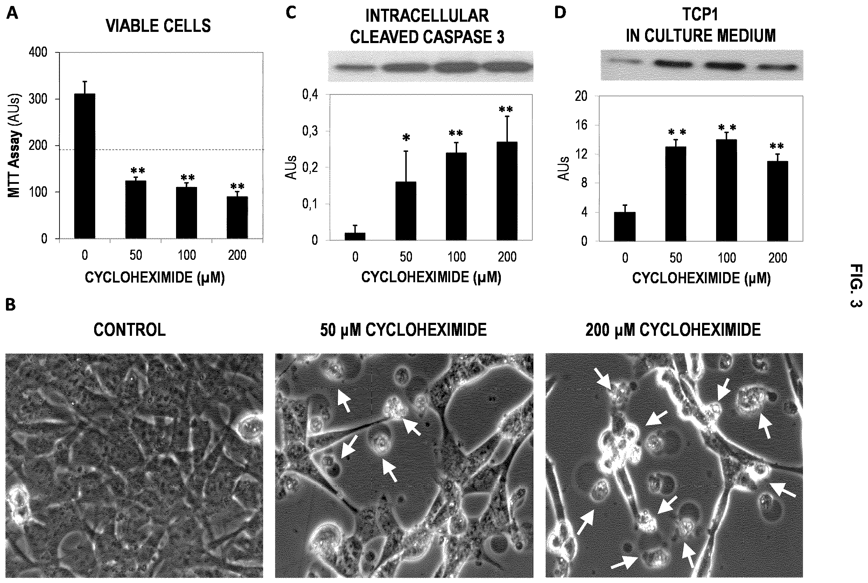

**Figure 3****. Cycloheximide also induces an apoptotic phenotype and TCP1-*eta* release, in a concentration-dependent manner.** MCT cells treated with 0 (control), 50, 100, and 200 µM cycloheximide for 18 hours. At the end of the experiment, (A) an MTT-based proliferation/viability was performed and (B) the death phenotype was determined by light microscopy, for the presence or absence of cell dismantling into apoptotic bodies (representative images are shown, 400x). The dotted line marks the starting point (time = 0 h). White arrows: apoptotic cells. (C) Representative images of Western blot analysis after 18h of treatment with cycloheximide of active caspase 3 (p19 fragment; n=2); and (D) TCP1-*eta* in the culture medium (n=3). And densitometry quantification. Data represent the average ± SD; *, p<0.05; ** p<0.01 with respect the control.

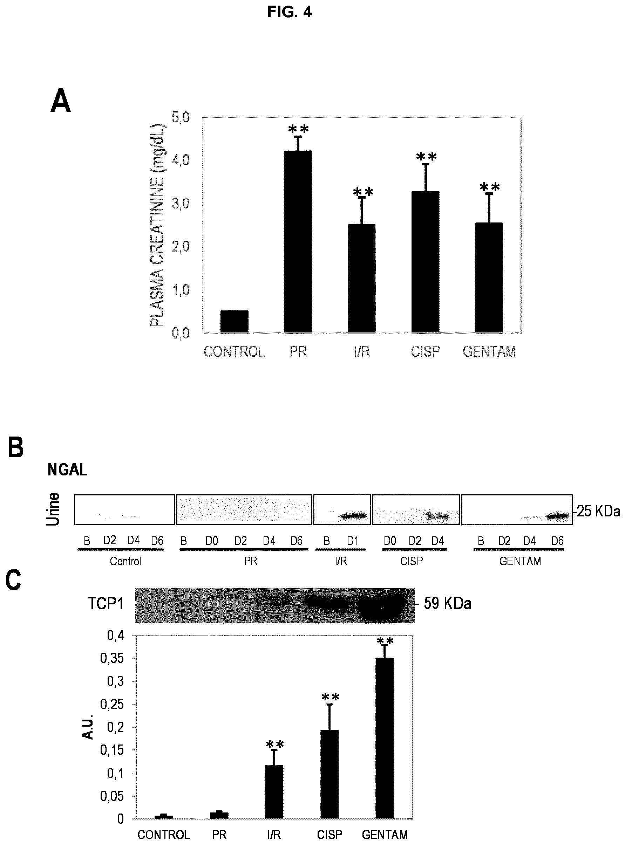

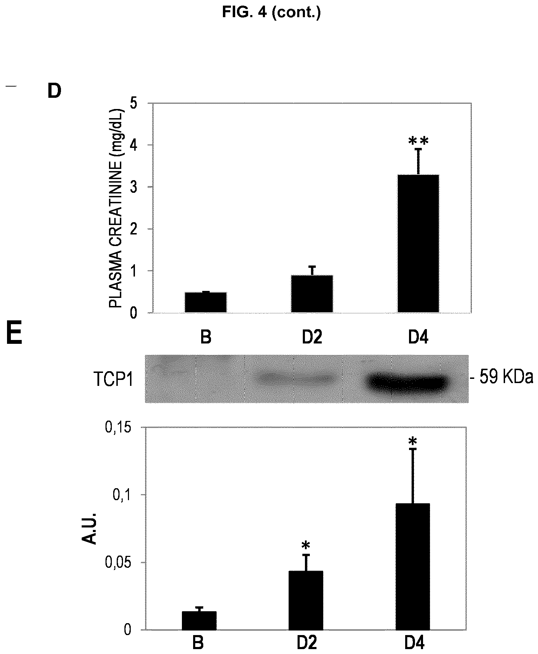

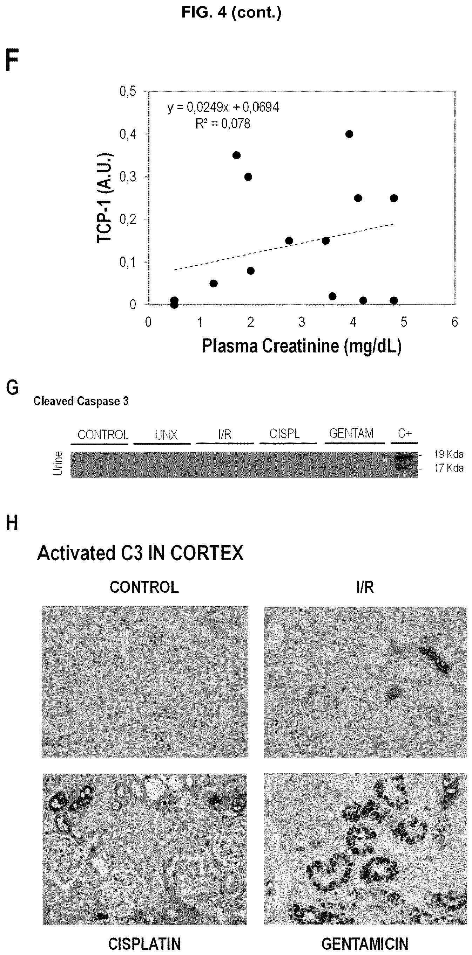

**Figure 4****. Urinary TCP1-*eta* is increased in different rat models of intrinsic AKI.** (A) Plasma creatinine, (B) representative image of Western blot analysis of urinary neutrophil gelatinase-associated lipocalin (NGAL) levels, and (C) urinary TCP1-*eta* levels of rats with different types of AKI. (D) Time course of plasma creatinine and (E) representative image of Western blot analysis of urinary TCP1-*eta* levels of rats treated with cisplatin. (F) Correlation analysis of urinary TCP1-*eta* and Crₚₗ of rats with different types of renal damage. (G) Representative image of Western blot analysis of urinary active caspase 3 in rat models of AKI. (H) Representative images (400x) of active caspase 3 immunostaining in the renal cortex of tissue specimens from I/R, cisplatin and gentamicin rats. AU, arbitrary units; B, basal point; D2 and D4, 2 and 4 days, respectively, after cisplatin administration. *, p<0.05; ** p<0.01 with respect the control. PR, prerenal; I/R, ischemia/reperfusion; CISP, cisplatin; GENTAM, gentamicin.

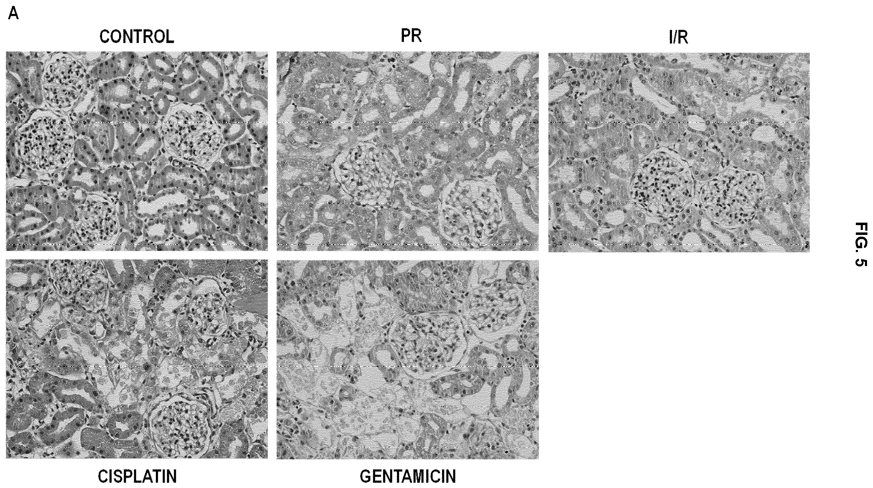

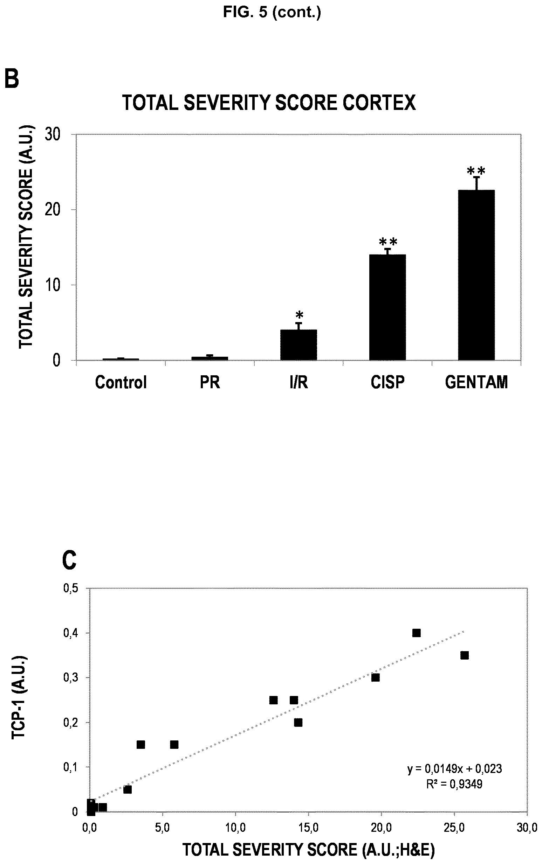

**Figure 5****. Urinary TCP1-*eta* correlates with the level of renal cortical damage.** (A) Representative images of the cortical area (400x) from kidney specimens stained with hematoxylin and eosin from control, prerenal (PR), ischemia/reperfusion (I/R), cisplatin (CISP) and gentamicin (GENTAM) rats. (B) Graphical representation of cortical damage severity score. (C) Correlation between urinary TCP1-*eta* levels and total severity score of cortical damage. AU, arbitrary units. * p <0.05 respect to control; ** p<0.01 with respect the control group.

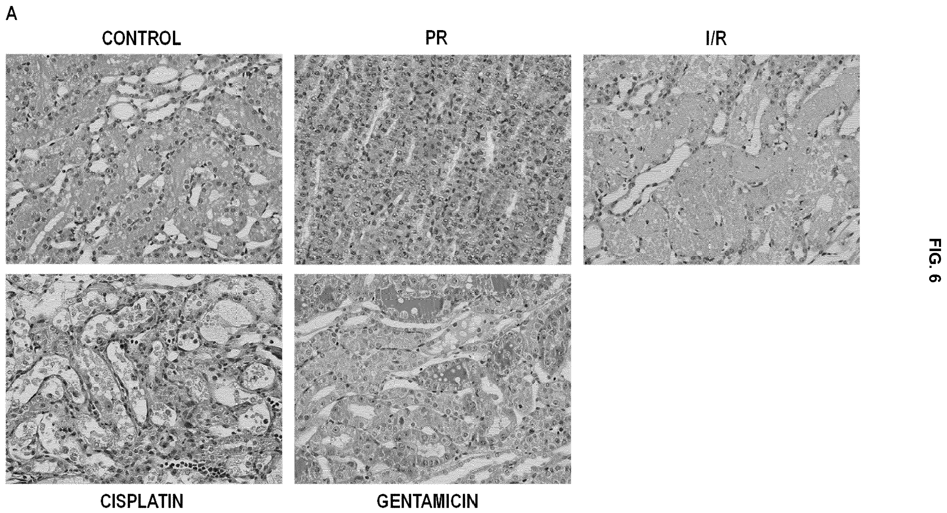

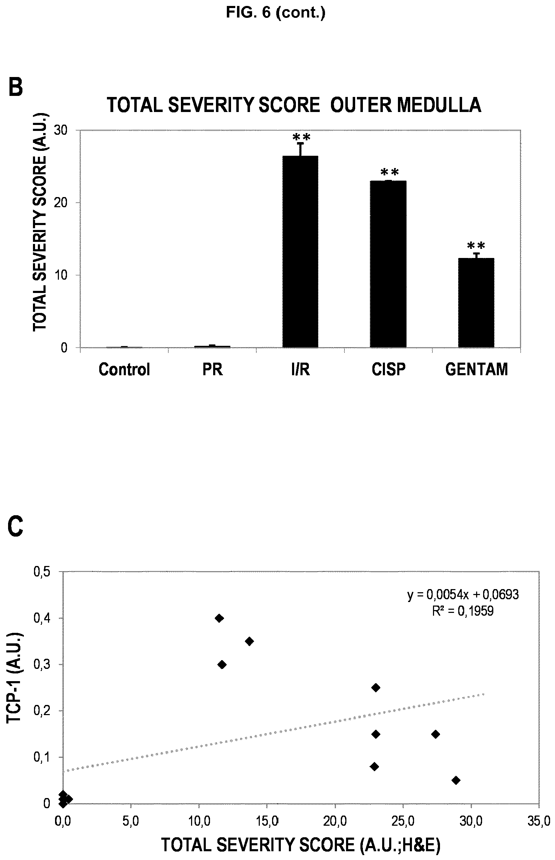

**Figure 6****. Urinary TCP1-*eta* does not correlate with the level of medullary damage.** (A) Representative images of the outer medullary area (400x) from kidney specimens stained with hematoxylin and eosin from control, prerenal (PR), ischemia/reperfusion (I/R), cisplatin (CISP) and gentamicin (GENTAM) rats. (B) Graphical representation of outer medulla damage severity score. (C) Correlation between urinary TCP1-*eta* levels and total severity score of outer medulla damage. AU, arbitrary units. * p <0.05 respect to control; ** p<0.01 with respect the control group.

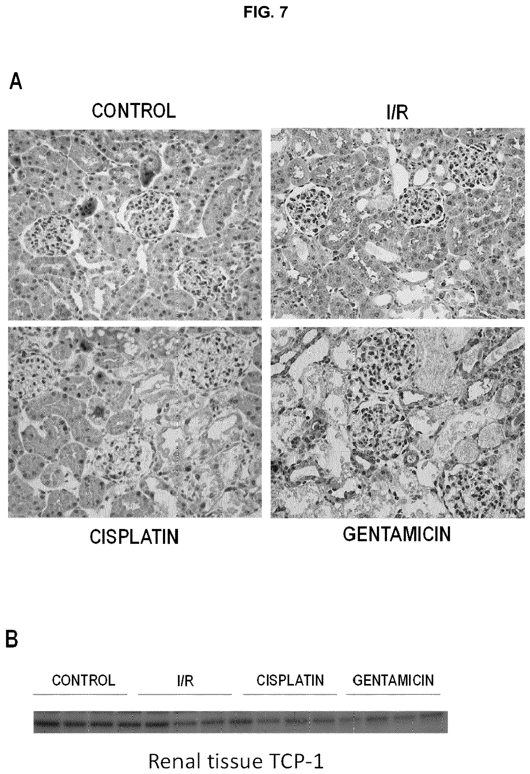

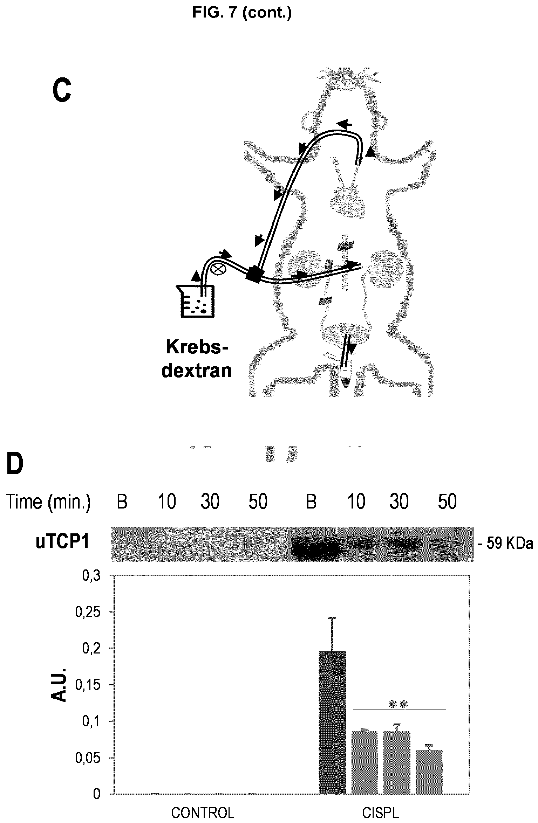

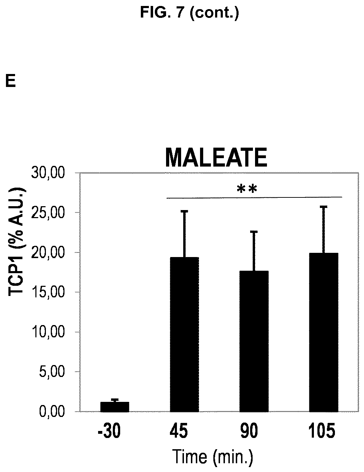

**Figure 7****. Urinary TCP1-*eta* partly comes from the blood and partly from the renal tissue.** (A) Representative images (400x) of the cortical area immunostained for TCP1-*eta* from control, ischemia/reperfusion (I/R), cisplatin and gentamicin. (B) Representative image of Western blot analysis of renal tissue TCP1-*eta* levels of rats with different types of AKI. (C) Schematic representation of the experimental set up used for *in situ* kidney perfusion with Krebs-dextran solution, and urine collection from the bladder. (D) Representative image of Western blot analysis of TCP1-*eta* levels and the corresponding densitometry quantification, in the urine of control and AKI rats during *in situ* renal perfusion. Data represent the average ± SEM. AU, arbitrary units, B, basal point; ** p<0.01 vs. basal point in its group. (E) Time-course of TCP1-*eta* urinary level in rats treated intravenously with a bolus of sodium maleate. Data represent the mean ± SEM. AU, arbitrary units; **p<0.001.

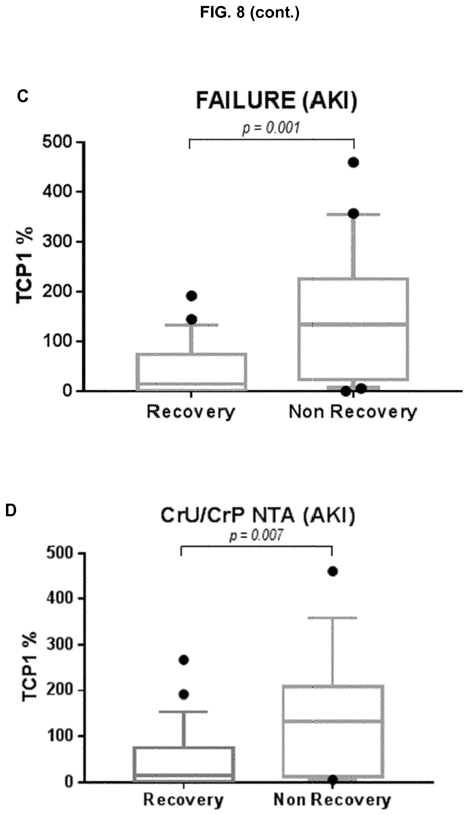

**Figure 8****. Urinary TCP1-*eta* during AKI predicts patient outcome.** (A) Representative image of Western blot analysis of urinary TCP1-*eta* levels in each study group, and the corresponding densitometry quantification. Urinary TCP1-*eta* levels in patients with favourable outcome (i.e. total recovery from AKI; Recovery) and with unfavourable outcome (i.e. incomplete or no recovery; Non Recovery) in the whole population of AKI patients (B), in patients with failure AKI (according to the RIFLE scale (C), and in patients with intrinsic AKI (according to their Crᵤ/Cr_{PI} ratio) (D).

### EXAMPLES

### Example 1. Cisplatin induces apoptosis of MCT cells and a parallel shedding of TCP1-eta to the culture medium.

Cisplatin concentration-effect relationship was studied in MCT cells cultured in medium without FBS. Serum withdrawal was implemented in order to avoid interference of serum proteins with MCT-secreted proteins. MCT cells responded to cisplatin in a very similar way than in the presence of serum with respect to cytotoxic potency and efficacy (comparative data not shown). After 18 hours of treatment, cisplatin reduced cell viability and increased the number of apoptotic cells (Figure 1a-c), caspase (DEVDase) activity (Figure 1d) in the absence of plasmalemmal rupture (i.e. no LDH release; Figure 1e), and internucleosomal DNA fragmentation (Figure 1f), which are hallmarks of apoptosis, in a concentration-dependent manner.

The protein content of the culture medium of MCT cells treated for 18 h with 0-30 µM cisplatin was studied by 2D proteomic analysis. Secretomes were very similar in all conditions (Figure 2a). However, a specific protein-spot consistently increased in the culture medium after treatment with cisplatin (Figure 2a) was unambiguously identified by mass spectrometry as TCP1-*eta*. Western blot analysis confirmed that the level of this protein increased with increasing concentrations of cisplatin (Figure 2b). RT-qPCR experiments demonstrated progressive TCP1-*eta* gene induction with increasing concentrations of the drug (Figure 2c, left panel), in close relation with secretion. Whereas the expression of the housekeeping gene ribosomal protein S13 (RPS13) was not modified (Figure 2c, right panel). TCP1-*eta* did not accumulate inside the cells, as revealed by Western blot analysis of cell extracts (Figure 2d), indicating that the excessive expression of this protein was evacuated off the cells. Other apoptosis-related proteins, such as t-gelsolin (a proteolytic fragment of gelsolin generated by caspases) were not detected in the culture medium (Figure 2e).

### Example 2. TCP1-eta is also a marker of cycloheximide cytotoxicity.

To study whether increased TCP1-*eta* net secretion also occurs for other cytotoxic stimuli, MCT cells were also treated with cycloheximide (50-800 µM) or vehicle (as control) for 18 h. Cycloheximide i) reduces cell viability (Figure 3a); ii) induces apoptosis, evidenced by detection of cells with apoptotic phenotype (Figure 3b) and executioner caspase 3 activation (Figure 3c); and also iii) increases TCP1-*eta* secretion to the culture medium (Figure 3d).

### Example 3. TCP1-eta is differentially elevated in the urine of rats with ATN, correlating with the degree of renal cortical injury.

Different rat models of AKI with distinct renal histological damage patterns were used to test whether secreted TCP1-*eta* reaches the tubular lumen and appears in the urine as a marker of tubular damage, namely ischemia/reperfusion injury, cisplatin-induced AKI, gentamicin-induced AKI, and pre-renal AKI. Plasma creatinine significantly increases in all models (Figure 4a), whereas urinary NGAL (Figure 4b) and TCP1-eta (Figure 4c) only in the three models of ATN (i.e., I/R, cisplatin and gentamicin), progressively with increasing damage (Figures 4d and 4e). Consequently, there is no correlation between the urinary excretion of TCP1-*eta* and the extent of functional damage measured as increments in Crpl (Figure 4f). In contrast, a signature marker of apoptosis (i.e. cleaved caspase 3) is not excreted with the urine despite being highly expressed in the kidneys (Figures 4g and 4h).

The three ATN models show characteristic patterns of damage, with ischemia affecting mostly the outer medullary strip, cisplatin both the cortex and the outer medulla, and gentamicin mostly the cortex and, to a lesser extent, the medulla. Renal histology and renal damage scores (Figures 5 and 6) reveal that the level of urinary TCP1-*eta* tightly correlates with the level of cortical but not of outer medullary damage.

Congruently with *in vitro* studies, no accumulation of TCP1-*eta* is detected in kidney cells by immunohistochemistry (Figure 7a) and Western blot (Figure 7b) in any of the ATN groups. *In situ* renal perfusion experiments reveal that, when kidneys are perfused with Krebs-dextran solution, a part of the urinary TCP1-*eta* disappears from the urine, but another part remains excreted with the urine (Figures 7c and 7d). These results indicate that a part of the urinary TCP1-*eta* comes from the blood and ends up in the urine because of an altered renal handling (most probably deficient reabsorption); and another part is directly shed from renal cells (likely tubule cells) to the urine. TCP1-*eta* seems to be normally filtered and mostly reabsorbed in the proximal tubule, as deduced from two facts: i) in control conditions, no significant, or little, TCP1-*eta* is detected in the urine; and ii) in normal rats treated with sodium maleate, an inhibitor of proximal tubule reabsorption, TCP1-*eta* is excreted with the urine (Figure 7e).

### Example 4. High levels of urinary TCP1-eta are associated to bad prognosis in AKI patients.

Urinary TCP1-*eta* was also investigated in patients in the context of AKI. A cohort of etiologically heterogeneous patients referred to the Nephrology Service with AKI symptomatology were included (Table 1).

**Table 1. Patient characteristics.**

| | **CONTROL** | **DISEASE CONTROL** | **AKI** |
|---|---|---|---|
| **N** | 12 | 6 | 68 |
| **GENDER (F/M)** | 11/1 | 0/6 | 23/63 |
| **AGE** | 49 ± 4 | 58 ± 4 | 63 ± 2 |
| **CrP (mg/dL)** | 0.7 ± 0.03 | 1.4 ± 0.18 | 5.8 ± 0.42 |
| **C-G** | 132.6 ± 7.15 | 64.7 ± 8.18 | 19.9 ± 4.34 |

They were catalogued as recovery or non-recovery depending on whether Crpl returned to values prior to the AKI episode or not, respectively. Healthy controls and disease controls (without AKI) were also studied. The urinary level of TCP1-*eta* was measured at admission. Taken as a whole, the cohort of AKI patients had higher levels of the urinary marker than healthy and disease controls (Figure 8a). However, there was a wide spectrum of urinary TCP1-*eta* values within AKI patients ranging from non-detectable to high levels. Controls posed the normal range of variability of urinary TCP1-*eta* levels, and the range of non-specificity (i.e. non-AKI-related). Interestingly, urinary TCP1-*eta* was significantly higher during AKI in patients who did not eventually recover, compared to those who completely did. This difference was statistically significant in the whole cohort (Figure 8b); and highly significant among patients at "failure" stage (according to the RIFLE scale; Figure 8c), and among those with intrinsic AKI (according to their Crpl/Cru ratio; Figure 8d). Logistic regression analysis showed good correlation between the urinary level of TCP1-*eta* and AKI evolution (i.e. full recovery or non-recovery) in the whole cohort, in failure AKI patients and in intrinsic AKI patients (Table 2).

**Table 2. Parameters of logistic regression analysis for data from B, C and D.**

| **Logistic regression** | **TOTAL (AKI)** | **FAILURE (AKI)** | **CrU/CrP NTA (AKI)** |
|---|---|---|---|
| B (P value) | 0.008 (0.014) | 0.012 (0.006) | 0.009 (0.020) |
| constant | -0.962 (0.01) | -1.222 (0.007) | -1.368 (0.004) |

### Example 5. Conclusions.

As a reasonable hypothesis, proteins found differentially in the supernatant of tubule cells cultured *in vitro* with cytotoxic drugs, might be eventually found also in the urine of animals treated with them. Our results show that tubule cells exposed to cisplatin handle TCP1-*eta* differently, by increasing outflow or decreasing reuptake, which results in higher amounts of this protein in the extracellular compartment. Increased outflow may be determined by the increased transcription, as no extra TCP1-*eta* accumulates inside the cells. The increased extracellular level of TCP1-*eta* seems a rather specific event, because control and cisplatin proteomes of the culture medium are quite similar. This is consistent with apoptotic cells and apoptotic bodies being tight bound and securing the intracellular content. Accordingly, TCP1-*eta* has been identified herein as a marker of cytotoxicity *in vitro,* which enables apoptosis monitoring over time by sampling and analyzing culture media.

An implicit hypothesis was that if tubular cells secrete higher amounts of TCP1-*eta* to the extracellular space, and if this also happened *in vivo,* TCP1-eta might reach the ultrafiltrate flowing through the tubular lumen and be found in the urine. In fact, this seems to be the case. Urinary excretion of TCP1-*eta* is significantly increased in three rat models of intrinsic AKI. Moreover, the level of TCP1-*eta* urinary excretion correlates with the degree of renal cortical injury rather than with the level of renal dysfunction (measured as Crpl). Interestingly though, *in situ* kidney perfusion experiments indicate that a part of the TCP1-eta found in the urine in AKI results from direct shedding from kidney (likely tubular) cells; and another part from an altered tubular handling (likely decreased reuptake) of the filtered TCP1-*eta*. Altogether, our results suggest that damaged cortical tubules both shed TCP1-*eta* to the urine and are less effective at reabsorbing this protein, as they normally do when they are unaltered.

Importantly, urinary TCP1-*eta* clearly associates to AKI in patients, and also to worse prognosis both in the general cohort, and more robustly when combined with other markers that further sub-stratify the AKI population. Specifically, high levels of this marker associate to defective recovery from AKI in the whole cohort, and even more robustly within those patients with theoretically worse prognosis, namely patients with failure AKI, and those with renal AKI, suggesting a close relationship between renal cortical damage and bad prognosis. From a gross estimation of worse prognosis anticipated by AKI severity or AKI type (i.e. population-based risk analysis), to a finer prognosis based also on TCP1-*eta*, this invention allows to identify at the individual level who, among these patients, will and who will not recover from AKI (i.e. truly personalized risk analysis).

In short, a cell-based method has been used herein for biomarker discovery and identifies TCP1-*eta* as a urinary marker of renal cortical tubule injury, which appears to be associated to defective recovery from AKI in patients. This biomarker contributes to the pathophysiological diagnosis of AKI for a more accurate patient stratification and personalized handling.

### Example 6. Materials and methods.

**Reagents, cells, and protocols.** Where not otherwise indicated, reagents were purchased from Sigma (Madrid, Spain). A mouse cortical tubule cell line (MCT) was used for the study. Cells were maintained in RPMI 1640 medium (Gibco, Madrid, Spain) supplemented with 10% fetal bovine serum (FBS, Gibco, Madrid, Spain), 500 U/mL penicillin, 50 mg/mL streptomycin (Gibco, Madrid, Spain) and 1 mM L-glutamine. Cells were treated in medium without serum for 9 and 18 h with cisplatin (0-100 µM), or for 18 h with cycloheximide (0-800 µM). During the treatments, cells were photographed under live cell, light microscope (Axiovert 200M, Zeiss; and Nikon Eclipse TS100, Barcelona, Spain) for visual inspection. At the end, cell extracts were obtained in extraction buffer (25 mM PIPES, pH7; 25 mM KCI; 5 mM EGTA; 0,5% NP-40, and a mixture of protease inhibitors consisting of 1 mM phenylsulfonyl fluoride, 1 µg/mL leupeptine, and 1 µg/mL aprotinin). Protein content was determined with a commercial kit (Bio-Rad, Madrid, Spain) based in the Lowry method.

**MTT assay.** Viable cell number was comparatively estimated by incubating cell cultures with 0.5 mg/mL 3-[4,5-dimethylthiazol-2-yl]-2,5-diphrnyl tetrazolium bromide (MTT) for 4h. Then, 10% SDS in 0.01 M HCL was added in 1:1 (vol/vol) and left overnight at 37 °C. Finally, absorbance was measured at 570 nm as an index of viable cell number.

**Caspase activity.** Twenty micrograms of protein from cell extracts were incubated with 100 µL reaction buffer (50 mM HEPES, pH7.4; 100 mM NaCl; 1 mM EDTA; 0.1% 3-[3-(cholamidopropyl)-dimethylammonio]-1-propane sulfonate; 10% sucrose; and 5 mM DTT) containing 100 µM of the fluorogenic caspase substrate rhodamine 110, bis-(N-CBZ-L-aspartyl-L-glutamyl-L-valyl-L-aspartic acidamide (Z-DEVD)2Rh110; Anaspec, Fremont, CA, USA)) at 37 °C for 30 min in a Fluoroskan Ascent FL fluorometer (Thermo, Rockford, IL, USA), and fluorescence intensity was measured every 3 min at 538 nm, upon excitation at 485 nm. Caspase activity was calculated from the slope of the linear relationship between fluorescence intensity and time.
**Western blot.** 20 µL from each supernatant and 30 µg from each cell extract were separated by acrylamide electrophoresis. Proteins were transferred to an Immobilon-P Transfer Membrane (Millipore, Madrid, Spain) and incubated with anti TCP1-*eta* antibody (Novus Biologicals, Littleton, CO, USA), followed by horseradish peroxidase-conjugated secondary antibodies and chemiluminiscent detection (Immobilon Western Chemiluminescent HRP Substrate kit; Millipore, Madrid, Spain) with photographic films (Kodak, Madrid, Spain).

**DNA fragmentation.** DNA fragmentation was determined with the commercial kit Cell Death Detection Plus (Roche Diagnostics, Barcelona, Spain) in 10 µg of protein from each cell extract.

**LDH assay.** LDH was measured in 50 µL culture supernatants with a CytoTox 96 non-radiactive cytotoxicity assay (Promega, Barcelona, Spain).

**RT-qPCR analysis.** Total RNA was isolated using Nucleospin RNAII (Macherey-Nagel, Düren, Germany). First-strand cDNA was generated from 1 µg of total RNA using poly-dT as primer with the M-MLV reverse transcriptase (Promega, Barcelona, Spain). Real-time PCR (qPCR) was performed in triplicate. In a total reaction volume of 20 µL containing 1 µL of cDNA, 0.4 µM of each primer and 1x iQSybrGreen Supermix (Bio-Rad), with initial denaturation at 95 °C for 5 min followed by 40 cycles of 30 sec at 95 °C, and 30 sec at 65 °C, and finally, one cycle of 30 sec at 72 °C. Threshold cycle (Ct) values were obtained. The sequences of TCP1-eta used were: sense 5'-GCCGCTTCCAAGATGATGCCCA-3' (SEQ ID NO: 1); anti-sense 5'-CGGGGACCCAGGGTGGTTCT-3' (SEQ ID NO: 2); and RPS13: sense 5'-GATGCTAAATTCCGCCTGAT-3' (SEQ ID NO: 3); anti-sense 5'-TAGAGCAGAGGCTGTGGATG-3' (SEQ ID NO: 4). Standard curves were run for each transcript to assure exponential amplification and to rule out non-specific amplification. Changes in the expression level were calculated following normalization with RPS13. The reactions were run on an iQ5 Real-Time PCR detection system (Bio-Rad, Madrid, Spain).

**Proteomic studies.** The culture supernatant of MCT cells treated with cisplatin or vehicle (as controls) in serum free medium for 18h was analyzed by two-dimensional electrophoresis (2-DE). Proteins (200 µg) were precipitated, isoelectrically focused (500-8,000 V) through 18-cm long immobilized pH gradient (IPG) strips, pH 3-11NL (GE Healthcare, Madrid, Spain), further separated in 12% SDS-polyacrylamide gels, fixed and stained with Sypro Ruby (Molecular Probes, Barcelona, Spain). The spots of interest were analyzed with the Image Master Platinum software (GE Healthcare, Madrid, Spain) and in-gel digested with porcine trypsin (Promega, Barcelona, Spain). Tryptic peptides were analyzed by MALDI-TOF on an Autoflex III instrument (Bruker Daltonics, Madrid, Spain). Protein identification was performed with the MASCOT software. In all protein identifications, the probability scores were greater than the score fixed by MASCOT as significant with a p-value lower than 0.05.

**Rat models of AKI.** Male Wistar rats (220-240 g) were housed under controlled experimental conditions and allowed free access to regular chow and water. Rats were divided into 6 groups (n=5 per group): (1) control group (control) saline solution; (2) ischemia-reperfusion group (I/R), one hour of warm renal ischemia plus contralateral nephrectomy; (3) cisplatin group (CISP), a single i.p. administration of 5 mg/kg cisplatin; (4) gentamicin group (GENTAM), receiving gentamicin i.p. (150 mg/kg per day for 6 days); (5) pre-renal group (PR), receiving ibuprofen (400 mg/kg plus trandolapril 0,7 mg/kg v.o. during ten days and on the fourth day, treated also with furosemide 20 mg/kg i.p. per day during 6 days); (6) maleate group (Maleate, n=4): a single i.v. injection of sodium maleate (400 mg/kg). Rats were allocated in metabolic cages to obtain 24-hour, individual urine samples. Blood was drawn with a needle from the tail vein, and plasma was obtained by centrifugation and, at the end of the treatments, kidneys were perfused with heparinized saline and immediately dissected. Half of the kidney was fixed in paraformaldehyde for histological studies, and the rest was frozen at -80 °C for biochemical analysis.

**Renal function.** Renal function was monitored by means of plasma creatinine and urea and urinary protein concentration, which were determined with commercial colorimetric kits (Quantichrom Creatinine and Quantichrom Urea Assay Kits, BioAssay Systems, Hayward, CA, USA), according to the manufacturer's instructions.

**Renal histopathology.** Paraformaldehyde-fixed tissue samples were immersed in paraffin, cut into 5mm-thick slices and stained with hematoxylin and eosin. Tissue slices were also immunostained with anti TCP1-*eta* antibody (Novus Biologicals, Littleton, CO, USA) and anti-caspase-3 antibody (Cell Signaling, Danvers, MA, USA); followed by horseradish peroxidase conjugated secondary antibodies.

*In situ* **renal perfusion.** Male Wistar rats (220-240 g) were divided into the following groups: (i) Control (n=6): Saline solution; (ii) Cisplatin (CISPL 5, n=6): A single i.p. injection of 5 mg/kg cisplatin. At the end of the experimental protocol or treatment, rats were anesthetized and an extracorporeal circuit for kidney perfusion was set up. The renal artery, vein and ureter of the right kidney were ligated. The renal artery and vein of the left kidney and the urinary bladder were cannulated. Oxygenated and warm (37 °C) Krebs-dextran was perfused through the renal artery at 3 mL/min. The effluent from the renal vein was collected. Urine fractions were collected from a catheter placed in the urinary bladder, starting before the perfusion with Krebs (when blood was still passing through the kidney), and during perfusion with Krebs for 60 minutes. As a control to discard experimental artefacts, kidneys were also perfused *in situ* with blood from the carotid artery, and urine was collected as above.

**Patients.** Urine samples were collected from 86 volunteers from the Nephrology Department in Hospital Universitario Marqués de Valdecilla (Santander, Spain): 68 patients had pre-renal or renal AKI; 6 were disease controls, and 12 healthy individuals. The hospital provided renal function and diagnosis data. Glomerular filtration rate was estimated with the Cockroft-Gault (C-G) equation.

**Statistical analysis.** Cell and animal data are represented as the mean ± SD or SEM of n experiments, as indicated in each case. Statistical comparisons were assessed by the one-way ANOVA analysis followed by the Bonferroni post-hoc test. Patients' data normality was checked with the D'Agostino & Pearson normality test. Non-parametric data were compared using the Mann-Whitney-U-Test (two-tailed). Box plot graphics display the median, quartiles and ranges (10-90 percentiles). Correlation analyses were performed using the Spearman correlation tests. Logistic regression analysis was used to study the predictive capacity of TCP1-*eta* on patient recovery from AKI. The GraphPad Prism 7.0 (GraphPad Software, San Diego, CA, USA) and the IBM SPSS Statistics 23 (IBM, Madrid, Spain) software were used. A p-value <0.05 was considered statistically significant.

## Claims

1. Use of the expression levels of urine TCP1-*eta* as a quantitative biomarker for the *in vitro* diagnosis of acute kidney injury.

2. Use of the expression levels of urine TCP1-*eta* as a quantitative biomarker for the *in vitro* prognosis and sub-classification of acute kidney injury patients in intrinsic acute kidney injury, preferably intrinsic acute kidney injury due to renal cortical tubule damage, associated to a bad prognosis.

3. Use according to claim 2, wherein the intrinsic acute kidney injury is caused by ischemia/reperfusion or a nephrotoxic agent.

4. Use of the expression levels of urine TCP1-*eta* as a quantitative biomarker for the *in vitro* monitoring of the response to a treatment in a patient suffering from acute kidney injury.

5. Use of the expression levels of TCP1-*eta* as a quantitative biomarker for the *in vitro* testing of the nephrotoxic activity of a compound.

6. Use according to any one of claims 1 to 5, wherein the expression levels of TCP1-eta are protein levels.

7. An *in vitro* method for the diagnosis of acute kidney injury in a subject, wherein said method comprises the following steps:
a. quantifying the amount of urine TCP1-*eta* protein in an isolated urine sample taken from a subject,
b. comparing the value obtained in step (a) to a standard value obtained from the quantification of the amount of urine TCP1-*eta* protein in a urine sample isolated from a healthy subject, and
c. assigning the subject of step (a) to the group of patients suffering from acute kidney injury when the value obtained after the quantification of step (a) is significantly higher than the standard value.

8. An *in vitro* method for the prognosis and sub-classification of patients suffering from acute kidney injury in intrinsic acute kidney injury, preferably intrinsic acute kidney injury due to renal cortical tubule damage, wherein said method comprises the following steps:
a. quantifying the amount of urine TCP1-*eta* protein in an isolated urine sample taken from a patient suffering from acute kidney injury,
b. comparing the value obtained in step (a) to a standard value obtained from the quantification of the amount of urine TCP1-*eta* protein in a urine sample isolated from a subject suffering from pre-renal acute kidney injury, and
c. assigning the patient of step (a) to the group of patients suffering from intrinsic acute kidney injury, preferably intrinsic acute kidney injury due to renal cortical tubule damage, associated to a bad prognosis, when the value obtained after the quantification of step (a) is significantly higher than the standard value.

9. The *in vitro* method according to claim 8, wherein the intrinsic acute kidney injury is caused by ischemia/reperfusion or a nephrotoxic agent.

10. An *in vitro* method for monitoring the response to a treatment in a patient suffering from acute kidney injury, wherein said method comprises the following steps:
a. quantifying the amount of urine TCP1-*eta* protein in an isolated urine sample taken from the patient prior to the administration of the treatment,
b. quantifying the amount of urine TCP1-*eta* protein in an isolated urine sample taken from the same patient after the administration of the treatment,
c. comparing the value obtained in step (a) and the value obtained in step (b), and
d. assigning the patient of step (a) to the group of patients showing a good response to the treatment when the value obtained after the quantification of step (b) is significantly lower than the value obtained after the quantification of step (a).

11. The *in vitro* method according to any of claims 7 to 10, wherein the subject or patient is a human.

12. An *in vitro* method for testing the nephrotoxic activity of a compound, wherein said method comprises the following steps:
a. *in vitro* culturing cells and quantifying the expression levels of TCP1-*eta* gene or the amount of TCP1-*eta* protein secreted to the culture medium prior to the administration of the compound to be tested,
b. administering the compound to be tested to the cells in culture of step (a),
c. quantifying the expression levels of TCP1-*eta* gene or the amount of TCP1-*eta* protein secreted to the culture medium after the administration of the compound to be tested in step (b),
d. comparing the value obtained in step (a) and the value obtained in step (c), and
e. identifying the compound as nephrotoxic when the value obtained after the quantification of step (c) is significantly higher than the value obtained after the quantification of step (a).

13. The method according to claim 12, wherein cells are cortical tubule cells.

14. The method according to claim 12 or 13, wherein cells are from human origin.

15. Use of a kit comprising primers, antibodies and/or probes specific for TCP1-eta for the *in vitro* diagnosis of acute kidney injury, for the *in vitro* prognosis and sub-classification of acute kidney injury patients in intrinsic acute kidney injury, preferably intrinsic acute kidney injury due to renal cortical tubule damage, associated to a bad prognosis, for the *in vitro* monitoring of the response to a treatment in a patient suffering from acute kidney injury and/or for the *in vitro* testing of the nephrotoxic activity of a compound.


SEQUENCE LISTING

<110> FUNDACION INSTITUTO DE ESTUDIOS DE CIENCIAS DE LA SALUD DE
      CASTILLA Y LEON (IECSCYL)
      UNIVERSIDAD DE SALAMANCA (USAL)

<120> METHOD FOR THE DIAGNOSIS, SUB-CLASSIFICATION AND PROGNOSIS OF
      ACUTE KIDNEY INJURY

<130> EP2796.4

<160> 4

<170> PatentIn version 3.5

<210> 1
<211> 22
<212> DNA
<213> Artificial Sequence

<220>
<223> Sense primer for TCP1-eta

<400> 1
gccgcttcca agatgatgcc ca                                              22


<210> 2
<211> 20
<212> DNA
<213> Artificial Sequence

<220>
<223> Anti-sense primer for TCP1-eta

<400> 2
cggggaccca gggtggttct                                                 20


<210> 3
<211> 20
<212> DNA
<213> Artificial Sequence

<220>
<223> Sense primer for RPS13

<400> 3
gatgctaaat tccgcctgat                                                 20


<210> 4
<211> 20
<212> DNA
<213> Artificial Sequence

<220>
<223> Anti-sense primer for RPS13

<400> 4
tagagcagag gctgtggatg                                                 20